# EUROPEAN PATENT APPLICATION

(11) **EP 2 450 034 A1**
(43) Date of publication of application: **09.05.2012**
(21) Application number: 10793497.8
(22) Date of filing: 01.07.2010
(51) Int. Cl.: A61K 9/50, A61K 35/74

(54) **FORMULATION BASED ON THE SYNTHESIS OF MICROSPHERES MADE FROM CROSS-LINKED NATURAL GELATINE, USED AS A CARRIER FOR STRAINS OF PROBIOTIC LACTOBACILLUS SPP. FOR TREATING SKIN WOUNDS AND/OR LESIONS**

(30) Priority: 01.07.2009 CL 15052009
(71) Applicant: Universidad De Concepcion, Concepción (CL)
(72) Inventor: CASTRO INOSTROZA, Erica, Concepción (CL); BORQUEZ YAÑEZ, Rodrigo, Concepción (CL); GONZALES RIQUELME, Margarita, Concepción (CL); KLATTENHOFF STOHR, Dietter, Concepción (CL)
(74) Representative: Carvajal y Urquijo, Isabel
(86) International application number: PCT/CL2010/000024
(87) International publication number: WO 2011/000122

(57) **Abstract**

The technology involves a probiotic formulation for topical use that comprises (1) microspheres with a matrix composition based on cross-linked natural gelatin, and (2) a viable probiotic strain of *Lactobacillus spp.* The particularity of this initiative is due mainly to the joint action of both components, indispensable for obtaining the desired effect. We seek to protect the process for creating the said microspheres, and their use treating lesions such as ulcers, wounds, burns and/or surgical wounds, infected tissue and chronic infections, in addition to their cosmetological and dermatological applications.

## Description

### Field of the Invention

This technology is oriented towards clinical use, specifically the treatment of patients with varying degrees of burns and different types of skin lesions. This invention involves a probiotic formulation based on natural gelatin microspheres and viable lactic acid-producing probiotic strains.

### State of the Art

Each year, almost 3,000 people suffer burns accidents in Chile, a number which is expected to rise in the adult population in the coming years.

Burn lesions are the traumas with the highest tendency to cause serious damage, frequently having permanent physiological consequences. Exposure of the skin to heat can lead to the denaturation of cellular metabolic processes, causing necrosis. This is determined by three critical factors related to thermal contact: intensity of heat, duration of exposure and tissue conductivity. Burns and trauma stimulate systemic inflammation, affect the functioning of polymorphonuclear neutrophils and depress the monocytic fagocitary system.

The damage that burn patients suffer to the skin, the main defence barrier of the body, causes dysfunction at an immunocompetent level, leaving the said patients defenceless against infection from organisms in the digestive tract, skin and environment. For many years antibiotics have been used to prevent infections and burns, yet when an antibiotic is used, it not only destroys normal flora, but also produces a bacterial overpopulation resistant to the drug.

The excessive use of antibiotics causes a destabilization of intestinal flora and alters the integrity of epithelial surfaces, it hinders the absorption and metabolism of nutrients and drugs and compromises resistance to infections. In addition, it causes an increase in microorganisms resistant to antibiotics, creating a greater public health problem.

Resistant bacteria can cause higher morbidity and death, especially in patients with serious illnesses, as well as immunosuppressed patients. Resistance to antimicrobial drugs is a problem for the community and for healthcare establishments, where agents causing intrahospital infections are acquired and where the transmission of bacteria is intensified, due to the high vulnerability of the population.

Furthermore, the use of drugs has an impact on wound healing, as it can interfere with scarring, affect cells, and alter the production of growth factors, cytokines and other components of the healing process. Patients with chronic wounds require pharmacological treatments with multiple medicines (antibiotics, corticosteroids, immunosuppressants, anticoagulants, cytotoxic and anti-inflammatory drugs, etc.) that can have a positive or negative impact on healing.

Materials have now been developed to improve the healing of wounds in a moist environment, unlike traditional healing materials, where the wound is left exposed to the environment, covered with textile materials (cotton gauze and other types of textiles), as well as ointments and creams.

Recently, more energy has been devoted to developing new alternatives and more effective agents in the treatment of wounds. It is therefore necessary to seek solutions involving either a reduction in the pharmaceutical element, an increase in its concentration in the affected area or effectiveness limited exclusively to the specific area, in order to achieve higher efficiency with minimal adverse consequences.

The surprising technology that we seek to protect will solve the aforementioned problems.

Various documents exist describing inventions that could be considered relevant to this initiative. There follows a discussion of the most relevant documents within this category:

American invention patent US4822534 protects a method for producing microspheres comprising a water slurry containing an immobilizing agent, a polysaccharide (alginates), and an insoluble substance, such as calcium citrate that causes gelation of the immobilizing agent. The water slurry is then contacted with a hydrophobic liquid, such as a vegetable oil under conditions leading to the formation of a dispersion of droplets of the water slurry in the hydrophobic liquid. If desired, this method can be adapted to produce microspheres containing immobilised material, such as microorganisms, animal cells or plant cells. This technology differs from our initiative in that it protects a method for producing microspheres, with the potential of their being subsequently adapted as a possible transport, a description which does not correspond to what we seek to protect.

Invention patent US6190591 of the United States Patent and Trademark Office protects a method for encapsulating and embedding a component that can be sensitive to heat destruction, readily oxidisable, or a pharmaceutically, biologically, or nutritionally active component with a high water binding capacity selected from the group of proteins and hydrocolloids; with at least one plasticiser and at least one release-rate controlling component, the said component being hydrophobic and selected from the group of fats, oils, waxes, fatty acids, emulsifiers, polyolefins, paraffins, etc. This invention patent essentially protects the composition of the corresponding microspheres, a significant difference from our technology, the protection of which has a different objective.

American patent application US2003012819 protects a method for preparing products containing moisture-sensitive materials, including biological materials such as proteins, peptides or live cells, comprising at least the following: (i) a coating liquid containing at least one active agent, a sugar polymer and a water soluble/miscible solvent; (ii) a quantity of microparticles comprising a water soluble gel that forms solid particles; (iii) a fluidizing system for said microparticles within a processing chamber or a suitable apparatus for forming a fluidised bed of said microparticles; (iv) spraying the said coating liquid onto the said fluidised bed to coat the said microparticles under saturated moisture conditions; and (v) a drying stage for the coated microparticles. Also described are compositions and uses. This technology differs significantly from our initiative, as its procedure is different from that which we seek to protect.

American patent application US2003152629 describes a multi-component controlled release system that can be incorporated into oral care, food and beverage, and nutraceutical products, as it effectively encapsulates active ingredients and sensory markers, as well as flavours and sweetening and refrigerating agents. This system contains various solid hydrophobic nanospheres that can be natural wax, synthetic wax, fat, glyceride, fatty acid, a fatty acid derivative and mixtures thereof. Each nanoparticle comprises a nucleus and an effective amount of an active agent, dispersed homogeneously within moisture-sensitive microparticles. This microparticle is formed of a moisture-sensitive matrix material and a bioadhesive material surrounding the said core, with the said moisture-sensitive matrix material comprising a water soluble synthetic polymer, water dispersible synthetic polymer, starch derivative, natural gum, polysaccharide, protein, hydrocolloid and mixtures thereof. The food and nutraceutical products can be in the form of vitamins, phytochemicals, antioxidants, minerals, microorganisms, prebiotics, probiotics, fungi, yeasts, enzymes, proteins, peptides, etc. In this case, both the production methods and the final use of the microparticles differ greatly from our technology, the novelty requirement of which is therefore not affected.

Patent application WO2003096823 to the World Intellectual Property Organization describes an edible oil-in-water emulsion containing live microorganisms which have good longevity in products. 30% to 75% of the weight of this emulsion is comprised of oil, water, live microorganisms and a preservation system, as well as an edible acid content in the aqueous continuous phase of the emulsion in the range of 0.1% - 3% by weight and with a pH at 20°C no lower than 4.0. The said microorganism is selected from bacteria that ferment lactic acid, lactic yeasts, or mixtures thereof. The bacteria are selected from *Lactobacillus bulgaricus, Lactobacillus casei, Lactobacillus acidophilus, Lactococcus lactis, Streptococcus thermophilus, Bifidobacterium bifidum, Bifidobacterium lingum* and mixtures thereof. This technology does not affect our novelty requirement, as the use and composition differ considerably from our product.

Patent application WO2005089564 to the World Intellectual Property Organization describes a method for producing water-in-oil products. The method comprises the following: admixing a hydrophobic component with a water-in-oil emulsion to form a water-in-oil product, where the said hydrophobic component comprises a probiotic -a viable microorganism selected from the group *Lactococcus, Streptococcus, Lactobacillus, Bifidobacterium,* among others- in a hydrophobic medium; the hydrophobic medium containing an unsaturated liquid oil and triglyceride, the latter preferably a monoester in dry form, ideally in powder form, that is added to the probiotic. This differs entirely from our initiative, as the microsphere production procedure and materials do not correspond to that which we seek to protect.

Invention patent application WO2005115341 to the World Intellectual Property Organization describes particles suitable for oral administration, with the said particles comprising a substantially indigestible polymer matrix containing the composition and a lipid that is dissoluble in the recipient. This composition can be dissolved, suspended or encapsulated in the lipid that is emulsified. The polymeric matrix contains a polysaccharide that can be starch, cellulose, chitin, chitosan, alginates, gum, etc., as well as a protein. This invention's composition contains a bioactive component that can be polypeptides, hormones, antibiotics etc., as well as microorganisms such as bacteria (*Bacillus sp., Lactobacillus sp., Alteromonas sp., Pseudomonas sp.,* etc.) and yeasts. The lipid is an oil selected from vegetable oils, fish oil, algae oil, microbial oil and combinations thereof. This technology, its use and the way it is administered differ completely from our initiative.

American invention patent US7201923 protects an encapsulated product comprising a substantially homogeneous mixture of: a plasticised matrix material; an encapsulant uniformly distributed in the said plasticised matrix material and selected from a pharmaceutical, nutraceutical, fragrance or biologically active component; a hydrophobic agent selected from the group of fats, oils, waxes, fatty acids and synthetic polymers for controlling the rate of release of the encapsulant. This encapsulant contains an active component, such as a live microorganism or an enzyme dissolved or dispersed in a liquid plasticiser, that is mixed with a plasticizable matrix material. The claims regarding the production of these microspheres are different from that which our initiative seeks to protect, therefore not affecting the novelty requirement.

In accordance with the above information, no previous document exists in the prior art containing the totality of our invention.

### Description of the Invention

The present technology involves a formulation for topical use that is useful for the treatment of various types of wounds, consisting of essentially two components: (1) microspheres with a matrix composition based on cross-linked natural gelatin, and (2) viable lactic acid-producing probiotic strains of *Lactobacillus spp.,* preferably *Lactobacillus acidophilus* LPV 31.

The particularity of this initiative is due mainly to the joint action of both components, indispensable for the desired effect. This effect can be used in the treatment of wounds, ulcers, burns and/or surgical wounds; and surprisingly even in infected tissue and chronic infections, which are problematic cases that have not been addressed properly by conventional technologies. In addition, it has a cosmetological and dermatological use, e.g. in the treatment of acne and other skin pathologies.

The production of the said microspheres is carried out using a water/oil emulsion (W/O), with the discontinuous phase being preferably, but not exclusively, an aqueous natural gelatin solution, and the continuous phase comprising an organic solvent that is preferably, but not exclusively, vaseline or vegetable oil. In addition to these components, the preparation must contain a viable probiotic strain, preferably a lactic acid-producing strain. The aqueous gelatin phase can contain, in addition, a cross-linking agent, a plasticiser and an emulsifier.

Natural gelatin is required in our formulation as a polymeric matrix with the capacity of forming thermoreversible gels, i.e. gels that upon reaching a certain temperature pass from a gel state to a liquid state, releasing the probiotic bacteria contained therein. The gelatin concentration used for the production process of the microspheres is closely related to the size of the microcapsules obtained, which varies in a range between approximately 5%-15% by weight, preferably 11 %.

The cross-linking agent used is preferably, but not exclusively, glutaraldehyde, which cross-links the polymer chains, through the union of the free amine functional groups. This agent is required in a concentration range between 1%-7% w/v, preferably 4%.

The plasticiser used in this technology is preferably glycerol, as it is a non-irritant compound with low toxicity, and mainly because it enables an increase in resistance, elasticity and flexibility of the microsphere. The range used for the formulation is 5%-15% v/v, preferably 10%.

The emulsifier is necessary to stabilise the particles of the dispersed phase in the dispersing medium, generating a balance between the aqueous and oil phases. The emulsifiers are a mixture of sorbitan derivatives or other emulsifiers, such as sorbitan monooleate and polyoxyethylene (20) sorbitan monooleate. Both are used in a range of 5%-15% v/v, preferably 7% and 8% v/v, respectively.

The production process of the said microspheres consists of two stages: (1) activation of the bacteria strains *Lactobacillus acidophilus* LPV 31, and (2) production of the gelatin microspheres using vaseline or vegetable oil as continuous phase. Once the microspheres have been obtained, the probiotic strain, in this case *Lactobacillus acidophilus* LPV 31, can be administered in two ways:
1°: Incorporating the microorganism into the said microspheres. The probiotic is not affected by the stress of the process to which it is subjected, and it retains its probiotic properties.
2°: Producing microspheres and microorganisms in separate processes and joining them together at the moment of administration when treating lesions such as burns, ulcers or wounds.

In the first alternative, in order to develop the hydrated probiotic biomass of the strain LPV 31, the latter is cultured in a medium based on whey permeate. After harvesting, it is centrifuged to obtain the bacterial pellet. This pellet is added to the aqueous gelatin solution in a proportion of 15%-20% v/v.

In terms of the microspheres, these can be subsequently subjected -with or without bacteria- to dehydration processes such as vacuum drying, lyophilization or spray drying, obtaining a dry product containing a moisture percentage lower than 10%, enabling its stability and reducing metabolic activity if the strain has been incorporated.

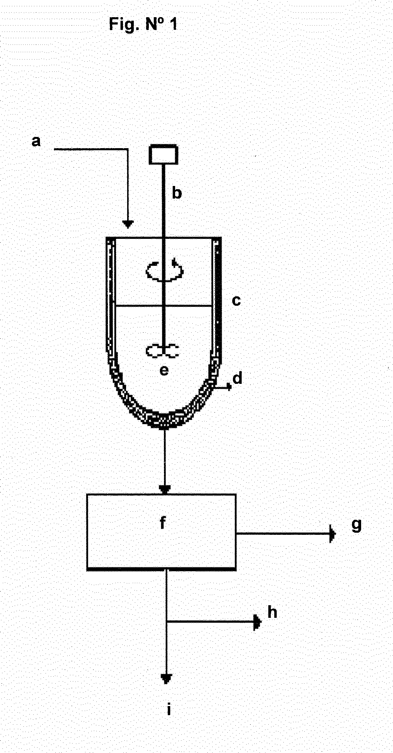

As mentioned previously, the production process of microspheres **(****Figure 1****)** is carried out using an emulsion comprising water and an organic medium, involving two immiscible liquid phases consisting of: a) an aqueous phase **(Figure 1a)** containing gelatin, water, cross-linking agent, biomass of the fresh probiotic strain (optional) and plasticiser; and b) a continuous organic phase of vaseline or vegetable oil **(Figure 1e).**

The system is composed of a glass container **(Figure 1c),** with an external shell **(Figure 1d),** making it possible to keep the emulsion at a constant temperature of 10°C in a thermostatic bath. The liquid is propelled through a mechanical turbine agitator **(Figure 1b),** producing a large amount of turbulence and shear within the liquid in the container in order to create the emulsion. Once the gelatin phase has been stabilised into a gel, this mixture is centrifuged **(Figure 1f), the** supernatant is eliminated and a sample is extracted to carry out a bacterial count on plates **(Figures 1g** and **1h,** respectively). This quality control procedure is carried out to determine the concentration of the probiotic strain, when added. Finally, microspheres **(Figure 1 i) of a** size over 2 µm and homogeneous size distribution are obtained.

The bacterial count shows the survival of the probiotic strain within the microspheres produced, with a total number of microorganisms in the range of 10⁶-10⁹ CFU/g.

In the second alternative, microsphere production is carried out through the previously described process, but without adding the bacterial biomass to the gelatin solution, and the bacterial suspension is dried in a spray dryer. In this case viability studies are also carried out to ensure that the strain does not suffer changes to its identity or morphology and that it is not contaminated during the process. Both products, cross-linked gelatin microspheres and dehydrated bacteria, are mixed at the moment of application to the patient's lesions.

During product application to the lesions, a synergetic effect is achieved, as the microsphere and the strain of *Lactobacillus acidophilus* (LPV31) potentiate each other to produce the desired effect. On the contrary, if these components are separate there is no effect whatsoever and there could even be adverse reactions on the part of the individual.

The efficacy of this technology was proven statistically using data obtained via a mouse model. This consisted in using the strain of *Lactobacillus acidophilus* LPV 31 incorporated into microspheres to treat mice with burn wounds that had been infected with a pathogenic microorganism commonly associated with this type of lesion, e.g. *Pseudomonas aeruginosa.* This procedure was carried out on various groups of animals, applying the microspheres either curatively or preventively.

Subsequently, tests were carried out on humans to validate this innovative initiative. This consisted of a clinical trial Phase 1, in two stages:
In **stage 1,** this new product, the strain Lactobacillus acidophilus LPV 31, incorporated into microspheres, was evaluated with healthy individuals to determine tolerance as well as potential adverse effects. These individuals were subjected to allergenic tests to evaluate their susceptibility to an allergic reaction. The first step was to determine the haematological evolution of the volunteers receiving this product. The next step was the identification of immunohaematological variations, observed in conjunction with the application of the probiotic microspheres. Finally, the type and frequency of adverse epidermic reactions observed with the application of the product were determined.

This trial made it possible to conclude that the probiotic components tested do not produce allergies, *in vivo* or *in vitro.*

Finally, in **stage 2,** this innovative technology was tested by administering it to patients with severe burns and chronic ulcers, producing significant results.

The totality of the results of this trial prove the effectiveness of these products, as the preventive application of the probiotic strain *Lactobacillus acidophilus* LPV 31 incorporated into microspheres indicates a clear inhibition of the pathogenic microorganism in the lesion area.

### Application Examples

### Example 1: Strain activation

In order to activate the probiotic strain *Lactobacillus acidophilus* LPV 31 the following steps were carried out:
a) A 100 µl sample of the strain *Lactobacillus acidophilus* LPV 31 was extracted and suspended in 4 ml of MRS medium, incubated in a stove at 37°C for 24 h.
b) 100 µl of the probiotic were transferred to 8 tubes with 4 ml of MRS medium and incubated in a stove at 37°C for 12 hours.
c) 4 volumetric flasks with 300 ml of whey permeate were inoculated with 600 µl of culture each, with an optical density between 5 and 6, and left for 14 h in a stove at 37°C at 90 rpm shaking frequency.
d) Subsequently, the contents of the volumetric flasks were transferred to centrifuge tubes and centrifuged at 3,500 rpm for 5 minutes, after which the supernatant was discarded and the pellet obtained.
e) Finally, the fresh bacterial mass of *Lactobacillus* was ready to be used in the microsphere production process.
f) If the biomass obtained is subjected to a dehydration process, it can be used as a component that is added at the moment of applying the microspheres to the lesion.

### Example 2: Microsphere production process

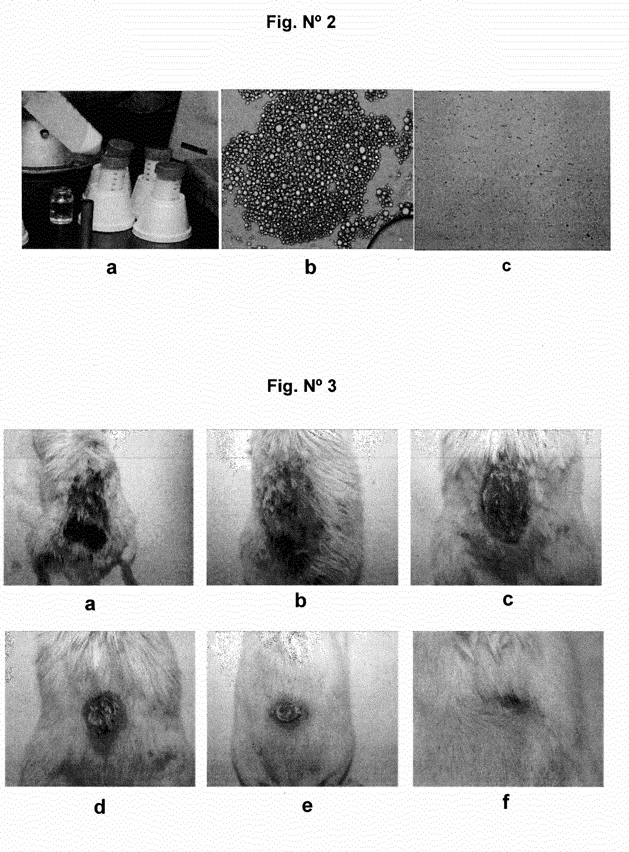

The production of the cross-linked gelatin microspheres consisted of the following stages:
a) An amount of gelatin equivalent to 11% w/v was weighed and subsequently added to a container with 40 ml of distilled water, shaken at room temperature.
b) The container with gelatin was placed in water, in a thermostatic bath at 55°C for 15 min approximately, until the gelatin dissolved completely.
c) Glutaraldehyde (0.006 M), previously saturated in toluene, was added at 4.2% w/v.
d) The emulsifier was incorporated: 7.5 ml of sorbitan monooleate and glycerol at 10%.
e) In a second container, 500 ml of vaseline were heated at 60°C in a thermostatic bath.
f) Maintaining constant shaking at 1500 rpm, the mixture of probiotic bacteria with the gelatin solution obtained in stage d was added to the container with vaseline. Subsequently, the mixture was kept at 10°C for 15 min.
g) Spheres, separated through centrifugation, were obtained from the emulsion resulting from stage f.
h) Finally, samples of the resulting microspheres were observed under an optical microscope in order to determine shape, average diameter and distribution. The microspheres obtained had an average size of 2.7 µm and are shown in Figures 2b and 2c (microspheres containing the probiotic strain).

### Example 3: Burn trial with mouse model

### Bacterial strain selected:

The strain selected was *Lactobacillus acidophilus* LPV 31, incorporated into microspheres. This strain was selected due to its probiotic properties, i.e. hydrophobicity, hydrogen peroxide production, as well as its capacity to adhere to and colonise inert supports. The pathogenic strain *Pseudomonas aeruginosa* P65-00 was also used, having been isolated from a surgical wound sample.

### Antibiotic:

Gentamicin at 0.1 % was used as a clinical treatment.

### Processing of bacterial strains:

The probiotic strain kept in milk supplemented with yeast extract (LEL) at -20°C was activated in an MRS culture medium, using 2 subcultures at 37°C, for 24 h and 14 h. For the LPV 31 strain a third inoculation was carried out in whey permeate for 12 h and resuspended until reaching a concentration of 10⁸ CFU/ml, in order to be finally incorporated into the microsphere support (ME). Microbiological counts were carried out throughout the test.

The *Pseudomonas aeruginosa* P65-00 strain was preserved in glycerol 50%, activated from a culture collection in a Luria-Bertanni medium and incubated at 37°C for 18 h. Subsequently, the strain was resuspended in cell saline solution until it reached a concentration of 5×10⁶ CFU/ml per application.

### Animals:

Male BALB/c mice between 7 and 8 weeks of age were used. The mice were kept in cages and fed *ad libitum* under controlled temperature (25°± 0.5°C), humidity and light conditions.

A total of 108 animals were used, separated into 4 experimental groups (n=18) and 3 control groups (n=12).

*Group 1:* Wound and prophylactic treatment with *Lactobacillus acidophilus* LPV 31 incorporated into microspheres for 3 days, as well as daily application of these for 2 weeks after infection with pathogens.

*Group 2:* Wound and prophylactic treatment with Gentamicin for 3 days, as well as daily application of Gentamicin for 2 weeks after infection with pathogens.

*Group 3:* Wound and curative treatment with *Lactobacillus acidophilus* LPV 31 incorporated into microspheres for 2 weeks after infection with pathogen.

*Group 4:* Wound and curative treatment with Gentamicin for 2 weeks after infection with pathogen.

*Group* 5: Wound and treatment with *Lactobacillus acidophilus* LPV 31 incorporated into microspheres for 2 weeks.

*Group* 6: Wound and treatment with Gentamicin for 2 weeks.

*Group* 7: Wound and infection with pathogen.

### Preparation of wound area:

The hair was removed from the back of the mice and each animal was anaesthetized via a subcutaneous injection of a 0.03 ml solution of Xylazine (PACIFOR)2%, Ketamine (10 µg/ml) and Acepromazine 1%, in a proportion of 4:3:1.

### Wound:

The back of each mouse was burnt with water at a temperature of 90°C for 10 seconds, producing a wound covering 10% of total body area.

### Inoculation:

Daily inoculations were carried out with the probiotic strain and the antibiotic in the wound area, in accordance with the groups previously described.

A single dose of the pathogenic strain P65-00 was administered in the experimental groups to induce infection with this strain.

### Hydration:

The mice were hydrated with 0.5 - 1 ml of phosphate buffered saline solution, administered intradermally.

### Sampling times:

One animal per group was sacrificed after 24 h, 48 h, 5 days, 10 days, 15 days and 28 days.

### Strain sampling and processing:

### Blood

At each sampling time, blood samples were obtained through a transversal cut to each mouse's tail. The blood was collected in Hematocrit tubes and a blood film was made for its subsequent immunological and haematological analysis. In addition, a cardiac puncture was carried out on each sacrificed animal, and the blood obtained was inoculated into selective media (MRS, TSA) to detect bacteremia. The samples were incubated at 37°C and analysed after 7 and 14 days.

### Tissue

Three and two animals, in the experimental and control groups, respectively, were sacrificed using cervical dislocation, and tissue from the wound area, liver and spleen was removed aseptically through a ventral incision. Each tissue sample was macerated in PBS buffer. 50 µl of each maceration were placed in MRS agar and TSA agar. The plates were incubated for 24 to 48h at 37°C. The plates to isolate the lactic strain were incubated in a microaerophilic environment. In order to identify bacterial colonies, the cultures derived from the organ maceration samples that tested positive were analysed using microscopic analysis, oxidase tests, catalase tests and biochemical tests, as appropriate. The results were expressed in CFU per gram of tissue.

The preventive application of the probiotic strain *Lactobacillus acidophilus* LPV 31 incorporated into microspheres indicated a clear inhibition of pathogenic microorganism growth in the wound area. This was established due to the fact that the pathogenic microorganism was not detected in the final sampling times, remaining viable only up to the first week of the trial. In the last sampling point where it was detected, the concentrations of the pathogen were close to 10⁵ CFU/g (Table 1, *group 1* until 5 days). On the other hand, in the groups treated preventively with an antibiotic (*group* 2), as well as the two curative groups (*groups 3 and 4,* respectively), the pathogenic microorganism was detected until the end of the trial, with concentrations similar to those found in the control group (*group 7*).

**Table 1**

| Groups | CFU/gram of tissue in wound area | | | | | |
|---|---|---|---|---|---|---|
| | 24 h | 48 h | 5 days | 10 days | 15 days | 28 days |
| Group 1 | 1.02_{*}10⁵ | 8.74_{*}10⁵ | 1.07_{*}10⁵ | NG | NG | NG |
| Group 2 | 9.68_{*}10⁴ | 1.07_{*}10⁸ | 3.57_{*}10⁸ | 4.09_{*}10⁷ | 2.82_{*}10⁵ | 9.97_{*}10⁷ |
| Group 3 | 1.07_{*}10⁸ | 1.84_{*}10⁸ | 2.66_{*}10⁸ | 3.66_{*}10⁸ | 1.16_{*}10⁷ | 2.33_{*}10⁷ |
| Group 4 | 1.39_{*}10⁷ | 2.78_{*}10⁹ | 2.58_{*}10⁷ | 7.41_{*}10⁸ | 3.39_{*}10⁶ | 4.50_{*}10⁷ |
| Group 5 | NG | NG | NG | NG | NG | NG |
| Group 6 | 1.01_{*}10⁶ | 8.81_{*}10⁸ | 1.62_{*}10⁷ | 6.60_{*}10⁸ | 1.20_{*}10⁷ | NG |

| | | | | | | |
|---|---|---|---|---|---|---|
| NG = No Growth | | | | | | |

Applications of the strain LPV 31 in microspheres as a treatment after infection were not effective in inhibiting or reducing the development of the pathogen, as the concentrations reached were similar to those of the control group, even remaining until 28 days, unlike the pathogen control group. The same results were observed with applications of the antibiotic, both preventively and as a treatment.

The probiotic strain was isolated from tissue from the wound area throughout the trial in groups 1 and 3 until day 15. The concentrations found were similar for both groups in the sampling times, with the highest concentrations being found after 24 and 40 hours; that is, concentrations close to 10⁵ CFU/g (Table 2).

**Table 2**

| Groups | CFU / gram of tissue in wound area | | | | | |
|---|---|---|---|---|---|---|
| | 24 h | 48 h | 5 days | 10 days | 15 days | 28 days |
| Group 1 | 1.84_{*}10⁵ | 7.92_{*}10⁴ | 9.54_{*}10² | 1.52_{*}10⁴ | 2.55_{*}10² | NG |
| Group 3 | NG | 5.44_{*}10⁴ | 8.11_{*}10² | 1.32_{*}10³ | 8.34_{*}10¹ | NG |
| Group 5 | 1.06_{*}10⁷ | 1.08_{*}10⁵ | 1.89_{*}10³ | 2.16_{*}10⁴ | NG | NG |
| Group 6 | NG | NG | NG | NG | NG | NG |

| | | | | | | |
|---|---|---|---|---|---|---|
| NG= No Growth | | | | | | |

Although the concentration of probiotic strain detected in the wound area was lower than the original inoculation count, it was sufficient to exert its inhibiting or immunomodulating effect.

These experiments were validated through splenic and hepatic tissue analyses, extracted from mice from the different groups. Microbiological responses and the related tendencies in tissue were different to those found in the hepatic and splenic tissue, indicating that the antibiotic permits the presence and active action of the pathogen subsequent to its inoculation.

According to tissue analyses carried out in the different groups of mice with the different treatments, **Figure 3** shows the evolution of the wound in group 1, where the animals were treated prophylactically with *Lactobacillus acidophilus* LPV 31 incorporated into microspheres and subsequently exposed to the pathogenic microorganism *Pseudomonas aeruginosa,* after which treatment with LPV 31 was continued. In this case it is possible to observe a surprising recovery of approximately 95% of the wound over time: 24 h, 48 h, 5 days, 10 days, 15 days and 28 days **(****Figures 3a, 3b, 3c, 3d, 3e, 3f****,** respectively).

### Example 4: Clinical trial Phase 1

### 4.1 Evaluation of microspheres: hypersensitivity reactions

### Patient selection

The following criteria were taken into account:
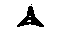 *Inclusion criteria:* healthy individuals without history of allergies, capable of giving informed consent.
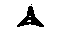 *Exclusion criteria:* individuals consuming corticosteroids and individuals suffering from chronic or immunocompromising illnesses.

### Sample size: 10 individuals of both genders.

*Clinical assessment:* prior to their entering the study, an immunologist from an external research centre applied a questionnaire to rule out active expressions of atopy and allergies. Individuals were only allowed to enter the study after the specialist had determined their viability as candidates.

The study was double-blind and lasted 21 days. It involved the application of microspheres with the probiotic strain LVP 31 and microspheres with the probiotic strain LLMO1. The strain applied first was referred to as A, while the second strain applied was referred to as B.

Initial sampling was carried out before administration of the product. This included the following analyses:
a) An immediate reaction (TDD) test with a preparation of 1 ml, in the back area, 24 hours before beginning the trial.
b) A blood sample from a vein for hemogramme, total IgE and PCR.
c) Subsequently, a test was carried out on the preparation, which was applied to the back area and removed 48 hours after its application.
d) In addition, a prick skin test was carried out in the back area, the results of which were revealed after 30 minutes.
e) Subsequently, each participant self-administered a daily dose of 1 ml of the sample, which was covered with a sterile swab in an area previously outlined on the left forearm, covering approximately 4x4 cm.
f) The second sampling of immunological and haematological parameters was carried out 21 days after the administration of the treatment.

### Clinical evaluation

Local clinical reactions, such as induration, erythema and reddening were evaluated.

In the prick test that measures type I hypersensitivity, mediated by class IgE antibodies, results were negative for all participants. The plaster tests measuring type IV, cell-mediated, hypersensitivity were also negative for all participants. A hemogramme was carried out for all 11 volunteers at time zero (T0). 6 of the volunteers presented increased levels of eosinophils (> 400/mm3), meaning that they were atopic and susceptible to the development of allergies. The 5 remaining patients did not present any of these characteristics (eosinophils< 400/mm3).

After the preparation had been applied to patients for 21 days (T1), eosinophils continued rising in general in the atopic individuals, whereas the remaining participants stayed within normal ranges. None of the participants developed allergies.

It can be concluded from this experience that the tested probiotic components do not produce allergies, *in vivo* or *in vitro.*

### 4.2 Clinical trial phase 1: burn patient

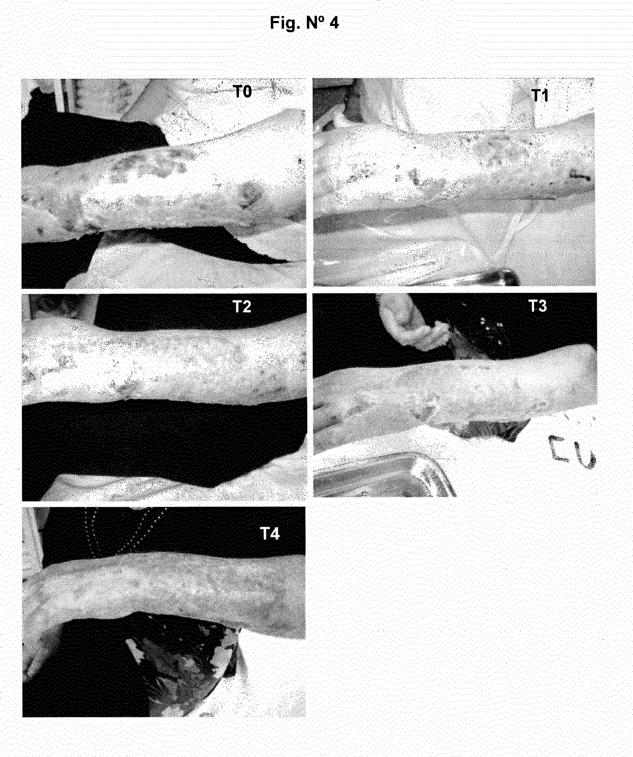

The following test was carried out on a patient with an extensive burn from 45 days prior, who voluntarily participated in the experience. The procedure for this test was the following: the microspheres and the probiotic strain LPV 31, that were initially separated, were incorporated together at the moment of application to the patient's lesion. The volunteer received an application of this preparation 3 times a week with a total of 12 applications. The evolution of the wound is shown in Figure 4, where T0 corresponds to the original 45-day lesion.

### 4.3 Clinical trial phase 2: patients with chronic ulcers

The effect of the microspheres containing a probiotic strain on the evolution of chronic ulcers of diabetic patients was evaluated.

This effect was determined through haematological analyses and through the observation of wound evolution together with the application, every 24 hours, of the bacterial strain *Lactobacillus acidophilus* LPV 31 incorporated into microspheres. Microbiological aspects of ulcer evolution were also evaluated, e.g. cultured pathogenic strains and molecular profiles of swabs from the wounds.

### Patient selection

The patients were individuals with ulcers more than 3 months old. These patients were randomly divided into groups and treated as follows:
- Conventional treatment administered by the health care centre.
- Application 3 times a week of the strain *Lactobacillus acidophilus* LPV 31 incorporated into the microspheres.

### Wound evolution

Local evaluation of the wounds included: location, dimensions (length, width, depth), base (epithelialised, granulated, necrotic), exudate (amount, colour, odour) and characteristics of the surrounding skin. A written and photographic record was kept during the trial.

### Wound assessment and sampling

Aetiological agents were identified on days 0, 5, 10, 15 and 30 using microbiological cultures. The presence or absence of *Lactobacillus sp., Pseudomonas aeruginosa, Staphylococcus aureus* and *Eschericia coli* was determined.

The presence of *Lactobacillus spp., Pseudomonas sp., and Staphylococcus aureus* was determined using molecular identification, specifically the polymerase chain reaction (PCR) technique.

### Haematological analysis

An initial hemogramme was carried out on the first day of treatment (day 0), and a blood sample was taken on the last day of the test (day 30), after which the haematological tests corresponding thereto were carried out.

### Cytological analysis

A sample of the inflammatory exudate from the wound was obtained before the ulcer was cleaned. This sample was extended and placed on a microscope slide for subsequent analysis. This was done on days 0, 5, 10, 15 and 30 during the treatment.

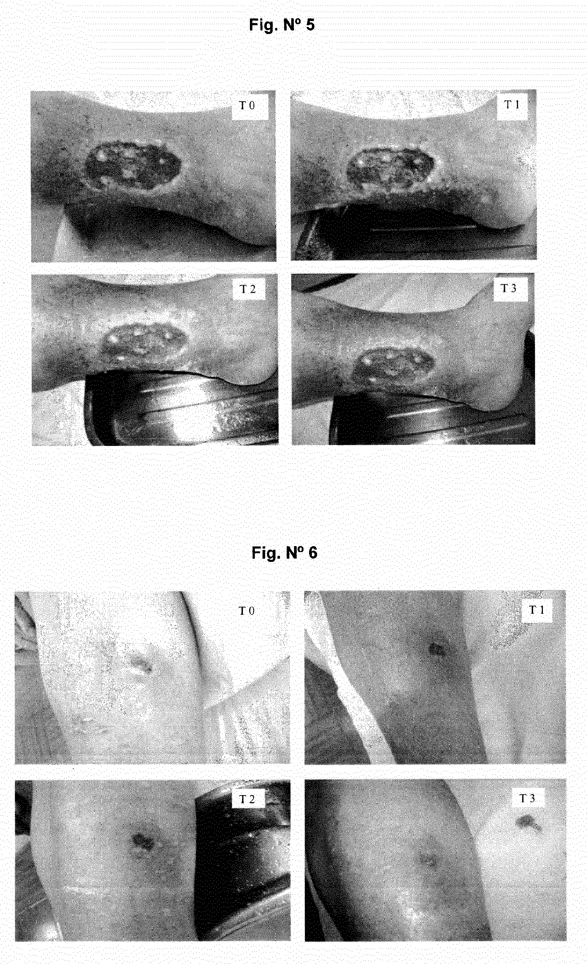

**Figure 5** shows the evolution of the wound of a 58-year-old volunteer, with a chronic varicose ulcer 5 years old, located on the right leg, the dimensions of which were 4 to 6 cm. This volunteer continued with normal treatment. The ulcer maintained the same dimensions and aspects. On the other hand, **Figure 6** shows the evolution of the wound of a 73-year-old diabetic volunteer with hypertension and a chronic ulcer 3 months old, located on the right leg, with a depth of 0-1 cm. This volunteer received the preparation of microspheres containing the strain LPV 31. In this case the evolution of the wound over time was evaluated and a significant improvement was observed.

**Table 3** shows the microbiological evolution of the wounds in the different cases treated with the microsphere preparation, as well as two control cases treated conventionally. It can be observed that patients treated with this new preparation show a reduction in microorganisms isolated from infected wounds, such as *P. aeruginosa, S. aureus* and *E*. *coli.*

**Table 3**

| | **Day 0** | **Day 30** | **Day 60** |
|---|---|---|---|
| Case 1 | *P. aeruginosa (-)* | *P. aeruginosa (+)* | *P. aeruginosa (-)* |
| Case 2 | *P. aeruginosa (*+*)* | *P. aeruginosa (+)* | *P. aeruginosa (-)* |
| | *E. coli (*+*)* | *E. coli (+)* | *E. coli* (+) |
| | *S. aureus (-)* | *S*. *aureus (+)* | S. *aureus (-)* |
| Case 3 | *S. aureus (+)* | *S. aureus (-)* | ----- |
| Case 4 | *E. coli* (+) | *E. coli (+)* | *E. coli (+)* |
| | *S. aureus (+)* | *S. aureus (-)* | *S. aureus (-)* |
| Control 1 | *E. coli (+)* | *E. coli (+)* | *E. coli (+)* |
| | *P. aeruginosa (-)* | *P. aeruginosa (-)* | *P. aeruginosa (+)* |
| Control 2 | *S. aureus (*+*)* | *S. aureus (+)* | *S. aureus (+)* |
| | *E. coli (-)* | *E. coli (+)* | *E*. coli *(+)* |

## Claims

1. A probiotic formulation for topical and/or dermatological use, suitable for the treatment of various types of wounds, **characterised by** at least two components: (1) microspheres with a matrix composition based on cross-linked natural gelatin with glutaraldehyde and a plasticiser, and (2) a viable lactic acid-producing probiotic strain of *Lactobacillus spp.,* preferably *Lactobacillus acidophilus* LPV 31.

2. A probiotic formulation for topical use, in accordance with claim 1, **characterised by** the fact that the microspheres with a matrix composition based on cross-linked natural gelatin contain the fresh viable probiotic strains.

3. A probiotic formulation for topical use, in accordance with claims 1 and 2, **characterised by** the fact that the concentration of the probiotic strain within the microspheres corresponds to a number of microorganisms within the range of 10⁶-10⁹ CFU/g.

4. A probiotic formulation for topical use, in accordance with claim 1, **characterised by** the fact that the microspheres with a matrix composition based on cross-linked natural gelatin and the dehydrated viable probiotic strain are separate additives that are mixed at the moment of administration.

5. A process for generating a probiotic formulation for topical and/or dermatological use, **characterised by** the fact that the said process consists of:
a) Activation of the viable lactic acid-producing probiotic strains of *Lactobacillus spp.,* preferably *Lactobacillus acidophilus* LPV 31, used fresh in the microsphere production process or subjected to a dehydration process through drying.
b) Production of microspheres using a water/oil emulsion, with a continuous phase and a discontinuous phase, comprising natural gelatin, a cross-linking agent, a plasticiser and an emulsifier.
c) The optional incorporation of the fresh viable probiotic strain.
d) Separation of the spheres produced through centrifugation.

6. A process to generate a probiotic formulation for topical use, in accordance with claim 5, **characterised by** the discontinuous phase comprising:
a) Preferably, but not exclusively, an aqueous natural gelatin solution in a range of 5%-15% in weight, preferably 11 %.
b) A cross-linking agent that is preferably, but not exclusively, glutaraldehyde, in a concentration range between 1 %-7% w/v, preferably 4% w/v.
c) A plasticiser, preferably glycerol, in a range between 5%-15% v/v, preferably 10%.
d) An emulsifier that comprises a mixture of sorbitan derivatives or other emulsifiers, preferably but not exclusively sorbitan monooleate and polyoxyethylene(20) sorbitan monooleate, both used in a range between 5%-15% v/v, preferably 7% and 8% v/v, respectively.

7. A process for creating the probiotic formulation for topical use, in accordance with claim 5, **characterised by** the fact that the continuous phase comprises an organic solvent, preferably but not exclusively vaseline or vegetable oil.

8. A use of the probiotic formulation, **characterised by** the fact that the said formulation, comprising microspheres with a matrix composition based on cross-linked natural gelatin and the probiotic *Lactobacillus spp.* strain, preferably *Lactobacillus acidophilus* LPV 31, is suitable for the treatment of burns, wounds, ulcers, surgical wounds, infected tissue and chronic infections, and cosmetology.
